# FASCICULE DE BREVET EUROPEEN

(11) **EP 1 532 927 B1**
(45) Date de publication et mention de la délivrance du brevet: **19.12.2007**
(21) Numéro de dépôt: 03292901.0
(22) Date de dépôt: 21.11.2003
(51) Int. Cl.: A61B 6/14, H05G 1/64, G01N 23/04, H04N 3/14, H03M 1/66

(54) **Appareil de radiologie dentaire**
Zahnärztliches Röntgengerät
Dental X-ray apparatus

(43) Date de publication de la demande: 25.05.2005
(73) Titulaire: Eastman Kodak Company, Rochester, New York 14650 (US)
(72) Inventeur: Boucly, Alain, 77124 Chauconin-Neufmontiers (FR); Inglese, Jean-Marc, 77437 Bussy-Saint-Georges (FR); Congy, Philippe, 77100 Meaux (FR)
(74) Mandataire: Petit, Maxime

(56) Documents cités:
- US-A- 5 519 437
- US-A- 5 572 566
- US-A- 5 604 781
- US-A- 5 677 940
- US-A- 5 933 107
- US-B1- 6 351 519

## Description

L'invention concerne le domaine de la radiologie dentaire.

On connaît des équipements de radiologie dentaire tels que celui décrit dans les brevets français FR 2 547 495 et européen n° 0 129 451. Le document US-A-5572566 décrit également toutes les caractéristiques du préambule de la revendication 1 de la présente demande.

De tels équipements comprennent une source de rayonnement X qui émet un rayonnement dirigé sur une dent située dans la bouche d'un patient et derrière laquelle se trouve un capteur intra-buccal qui reçoit le rayonnement ayant irradié la dent.

Ce capteur comporte :
- un scintillateur en entrée pour convertir le rayonnement X ayant irradié la dent en rayonnement visible,
- une plaque de fibres optiques pour transmettre le rayonnement visible converti à un détecteur à transfert de charge de type CCD, qui transforme le rayonnement visible converti en un signal électrique analogique, tout en absorbant le résidu de rayonnement X qui n'a pas été converti en rayonnement visible.

Le signal électrique est amplifié et transmis sous forme analogique à travers un long câble, jusqu'à un poste éloigné de traitement et de visualisation où le signal est numérisé et traité afin de produire une image qui est ensuite visualisée sur un écran.

Ce type d'équipement avec un détecteur à transfert de charge génère un rapport signal sur bruit (SNR) élevé, par exemple, de l'ordre de 60 dB.

On connaît également, d'après le brevet US 5,912,942, un type de détecteur de rayonnement X dans lequel le détecteur dit à pixels actifs et connu sous le nom de détecteur APS ("Active Pixel Sensor") utilise la technologie de fabrication CMOS.

Dans le brevet précité, l'équipement de radiologie qui y est décrit comprend :
- une source de rayonnement X irradiant un objet,
- un scintillateur qui convertit en lumière visible le rayonnement ayant irradié l'objet,
- une plaque de fibres optiques transmettant la lumière visible convertie jusqu'à une matrice à pixels actifs qui la transforme en signal électrique analogique.

On a pu constater que le détecteur CMOS procure un rapport signal sur bruit (SNR) de moins bonne qualité que celui du détecteur à transfert de charge.

A cet effet, on a d'ailleurs identifié plusieurs facteurs qui limitent le rapport signal sur bruit du détecteur CMOS.

Parmi ces facteurs, on trouve le courant d'obscurité qui peut être défini comme étant le courant électrique recueilli en sortie du détecteur lorsque ce dernier n'est exposé à aucun rayonnement X.

La présence du courant d'obscurité conduit à une dégradation du rapport signal sur bruit.

On notera que, dans la mesure où l'intensité du courant d'obscurité présente la particularité d'augmenter considérablement avec la température et comme le détecteur chauffe lors de son utilisation, il est conseillé de le refroidir et/ou de ne pas le faire fonctionner trop longtemps afin de ne pas dégrader davantage le rapport signal sur bruit.

Un deuxième facteur limitant est le coefficient de remplissage du détecteur.

Pour un détecteur à transfert de charge, le coefficient de remplissage est, en théorie, de 1, ce qui signifie que toute la surface du pixel est utilisée pour capturer le rayonnement et produire la charge électrique correspondante qui va contribuer à la formation de l'image de la dent irradiée.

Au contraire, dans un détecteur CMOS à pixels actifs, l'élément actif du pixel occupe une partie de la surface du pixel, sans toutefois contribuer à la capture du rayonnement.

Une partie du pixel ne contribuant pas au remplissage, c'est-à-dire ne contribuant pas à la conversion photon-électron, on a donc un coefficient de remplissage inférieur à 1, ce qui nuit à l'obtention d'un bon rapport signal sur bruit.

Un troisième facteur limitant résulte du fait qu'aujourd'hui on ne sait pas fabriquer en technologie CMOS une matrice de pixels actifs monolithique de grandes dimensions, typiquement de l'ordre de 20 x 30 mm qui sont les dimensions couramment utilisées pour les capteurs de radiologie dentaire.

Pour obtenir en technologie CMOS une matrice à pixels actifs de grande dimensions, il est nécessaire d'assembler entre elles par collage (technique connue en terminologie anglosaxonne sous le terme "stitching") plusieurs sous-matrices de dimensions inférieures.

L'hétérogénéité créée par une matrice ainsi obtenue contribue à dégrader le rapport signal sur bruit.

En outre, il convient de noter que les conditions spécifiques au domaine de la radiologie dentaire rendent particulièrement difficile la conception d'un appareil de radiologie dentaire avec un rapport signal sur bruit de très bonne qualité.

En particulier, dans la mesure où l'on expose des personnes à un rayonnement X, il convient d'utiliser des doses de rayonnement aussi faibles que possible et d'exposer ces personnes le moins longtemps possible à de tels rayonnements.

Dans d'autres domaines où l'on utilise un détecteur de rayonnement X en technologie CMOS, les doses de rayonnement X n'ont pas besoin d'être aussi faibles qu'en radiologie dentaire, ce qui permet d'avoir en sortie du détecteur un signal d'intensité plus élevé, et donc un meilleur rapport signal sur bruit.

Par ailleurs, l'une des particularités des capteurs de radiologie dentaire intra-buccaux tient à ce que le capteur qui est placé dans la bouche d'un patient doit être le moins volumineux possible pour limiter la gêne occasionnée au patient, ce qui impose de réduire le nombre de composants du capteur.

On notera que, dans un mode de réalisation préféré du détecteur de rayonnement X décrit dans le brevet US 5, 912,942, le détecteur comprend en lui-même un convertisseur analogique numérique afin de numériser sans tarder le signal de sortie analogique qui va être transmis à l'ordinateur distant.

Une telle conception du détecteur va à l'encontre de la miniaturisation nécessitée par une installation dans la bouche d'un patient.

De plus, l'introduction d'un convertisseur analogique numérique à côté d'une matrice à pixels actifs réalisée en technologie CMOS qui, elle, est un élément analogique, constitue une source de bruit supplémentaire qui, ajoutée à la contrainte d'une dose minimale de rayonnement X, contribue à dégrader le rapport signal sur bruit du détecteur.

On notera que d'autres sources de bruit non citées sont également susceptibles d'affecter le rapport signal sur bruit du détecteur.

De manière générale, il existe un besoin de nouveaux appareils de radiologie dentaire et de procédés de traitement de signal dans de tels appareils qui permettent d'améliorer le rapport signal sur bruit fourni par le détecteur de l'appareil.

Par ailleurs, on s'est également aperçu que l'utilisation d'appareils de radiologie dentaire existants donnait lieu à des problèmes d'hygiène qu'il serait souhaitable de résoudre, au moins dans une certaine mesure.

Ainsi, lorsque le dentiste équipé de gants d'intervention vient de placer derrière une dent, dans la bouche d'un patient, un capteur intra-buccal qui comprend l'un des types de détecteur visés plus haut, il doit rallumer le capteur et ensuite mettre en marche le générateur de rayons X.

Pour ce faire, il doit se rendre jusqu'à l'ordinateur situé à plusieurs mètres de distance, ce qui n'est déjà pas pratique, et cliquer ensuite sur une souris d'ordinateur pour déclencher le capteur et également le générateur de rayons X par l'intermédiaire d'une interface logicielle.

Or, à ce moment, le dentiste porte des gants qui sont déjà contaminés par la salive du patient, ce qui risque d'entraîner une contamination croisée lorsque le dentiste manipulera ultérieurement la souris d'ordinateur avec des gants imprégnés de la salive d'un autre patient.

Face à une telle situation, le dentiste doit alors, soit ôter ses gants avant de manipuler la souris, soit désinfecter celle-ci après utilisation, ce qui représente dans les deux cas des contraintes supplémentaires qui deviennent vite pesantes lorsqu'elles se répètent plusieurs dizaines de fois par jour.

D'autre part, il existe également un besoin de disposer d'un appareil de radiologie dentaire d'encombrement aussi réduit que possible, notamment au niveau du capteur intra-buccal et de l'électronique associée.

L'invention vise donc à remédier à au moins un des problèmes mentionnés ci-dessus.

Selon un premier aspect, l'invention concerne un appareil de radiologie dentaire, tel que défini par les revendications.

Selon cet aspect de l'invention, le module électronique comporte au moins un organe d'activation du détecteur et est suffisamment proche du capteur pour être manipulé par un dentiste lors de son intervention sur un patient et ainsi permettre, en sollicitant l'organe d'activation par la main, ou par le pied si le module est posé par terre, de rallumer le capteur aux fins d'une prise d'image d'une dent.

Dans la mesure où le module électronique équipé de l'organe d'activation est situé dans la sphère d'activité du dentiste, ceci lui évite d'avoir à se déplacer jusqu'à l'unité de traitement et de visualisation qui est en pratique éloignée de plusieurs mètres de ce dernier.

On évite ainsi par là même des problèmes de contamination croisée qui risqueraient de se poser si le dentiste devait se déplacer jusqu'à l'unité éloignée de traitement et de visualisation et manipuler un organe d'activation du capteur tel qu'une souris d'ordinateur via une interface logicielle.

Dans l'hypothèse où la souris d'ordinateur devait être désinfectée, on évite également cette opération supplémentaire de désinfection.

Par ailleurs, la proximité du module électronique par rapport au capteur permet de disposer d'une liaison filaire analogique relativement courte (généralement inférieure à 1 mètre), ce qui permet de ne pas dégrader le rapport signal sur bruit du détecteur.

L'organe d'activation permet également de télécommander certaines des fonctions que le dentiste pourrait avoir à utiliser immédiatement avant ou après la prise d'image, avec les mêmes contraintes d'hygiène. Parmi ces fonctions, citons de façon non exhaustive, le retournement d'image pour une orientation maxillaire/mandibulaire, ou le réglage des paramètres de visualisation comme le contraste et la luminosité.

Cet aspect de l'invention est particulièrement simple à mettre en oeuvre.

On notera que le boîtier a une forme générale allongée qui lui permet d'être facilement manipulé et, de façon additionnelle, nettoyé.

Le module électronique encapsulé a par ailleurs un poids et des dimensions qui sont adaptés pour permettre, lors de l'utilisation de l'appareil, le maintien du capteur dans la bouche d'un patient lorsque ledit module électronique encapsulé est suspendu audit capteur.

Selon une caractéristique, le module électronique est à une distance comprise entre 50 cm et 2 m du capteur.

Selon une caractéristique, le module électronique encapsulé est plus proche du capteur que de l'unité de traitement et de visualisation.

Selon une caractéristique, ledit au moins un organe d'activation est un bouton poussoir.

Selon une caractéristique, chaque liaison filaire est un câble.

Selon une caractéristique, chaque câble s'insérant à une de ses extrémités dans le boîtier, le module électronique est muni de dispositifs anti-traction qui sont chacun aptes à coopérer avec une extrémité de l'un des câbles de manière à empêcher le retrait du câble correspondant du boîtier sous faction d'une traction exercée sur ledit câble.

Ces dispositifs anti-traction permettent d'éviter l'utilisation de connecteurs électriques qui introduisent des faux contacts et risquent de se déconnecter intempestivement.

Selon une caractéristique, chaque câble comportant une gaine coaxiale à un faisceau de fils électriques, au droit de l'extrémité de chaque câble qui s'insère dans le boîtier la partie du faisceau de fils correspondant est solidarisée à un corps métallique d'anti-traction du dispositif anti-traction correspondant.

Selon une caractéristique, le module électronique se présente sous la forme d'un circuit imprimé de forme générale allongée le long d'un axe longitudinal et comportant, à chacune de deux extrémités opposées disposées longitudinalement, une découpe axiale ouverte vers l'extérieur du circuit pour loger suivant l'axe longitudinal un corps métallique d'anti-traction et la partie du faisceau de fils correspondant solidarisée et alignée, la découpe étant aménagée pour empêcher le retrait du corps suivant cet axe longitudinal.

Selon une caractéristique, chaque corps métallique d'anti-traction est pourvu d'éléments d'emboîtement disposés sur des faces opposées parallèles à la direction de la partie du faisceau de fils solidarisée au corps et qui coopèrent avec des éléments d'emboîtement complémentaires aménagés respectivement sur les bords opposés longitudinaux de la découpe correspondante.

Selon une caractéristique, chaque partie de chacun des faisceaux de fils solidarisée à un corps d'anti-traction est solidarisée à un fût cylindrique qui, d'une part, entoure cette dernière et, d'autre part, est solidaire du corps correspondant.

Selon une caractéristique, chaque partie de chacun des faisceaux de fils solidarisée à un corps d'anti-traction est soudée directement ou indirectement à ce dernier.

Selon une caractéristique, deux demi-coques métalliques sont agencées de part et d'autre du circuit imprimé et assemblées l'une avec l'autre de manière à emprisonner ledit circuit imprimé.

Ces demi-coques protègent mécaniquement le module électronique.

Selon une caractéristique, le boîtier comporte au moins deux parties en plastique formant couvercle et qui sont assemblées l'une avec l'autre de manière à encapsuler le module électronique.

Selon une caractéristique, le boîtier présente une surface extérieure désinfectable.

Selon une caractéristique, les formes de la surface extérieure sont de nature à ne pas favoriser l'incrustation de saletés.

Selon une caractéristique, la surface extérieure est étanche au ruissellement.

Selon une caractéristique, la liaison filaire entre le module électronique et l'unité de traitement et de visualisation est conforme à la norme USB2.0 ou peut également autoriser des vitesses de transmission supérieures à celles de la norme précitée.

Selon une caractéristique, le capteur comporte un convertisseur de rayonnement X qui est apte à convertir en rayonnement visible un rayonnement X ayant irradié une dent.

Selon une caractéristique, le détecteur biCMOS comprenant la matrice à pixels actifs est apte à transformer au moins une partie du rayonnement visible issu de la conversion du rayonnement X en au moins un signal de sortie électrique analogique.

D'autres caractéristiques et avantages apparaîtront au cours de la description qui va suivre, donnée uniquement à titre d'exemple non limitatif et faite en référence aux dessins annexés, sur lesquels :
- la figure 1a est une vue schématique partielle d'un appareil de radiologie dentaire selon l'invention lors d'une prise d'image d'une dent ;
- la figure 1b illustre une représentation classique d'un pixel actif utilisant trois transistors et une photodiode ;
- la figure 2 est une vue schématique partielle de l'appareil de radiologie dentaire selon l'invention et qui complète la représentation de la figure 1 a ;
- la figure 3 est une vue schématique générale du détecteur 22 représenté sur les figures 1 a et 2 ;
- la figure 4 est un circuit schématique illustrant la génération d'un signal de référence de sortie blackref du détecteur ;
- les figures 5 et 6 illustrent l'allure des signaux S-R avant et après le double échantillonnage qui est effectué dans le circuit 76 de la figure 3 ;
- les figures 7a et 7b représentent respectivement l'allure des signaux d'entrée et de sortie du circuit 79 de la figure 3 ;
- la figure 7c représente l'allure du signal d'échantillonnage Sample-clk ;
- la figure 7d représente schématiquement la conversion analogique numérique du signal vidéo à l'aide du signal d'échantillonnage ;
- les figures 7e à 7g illustrent successivement et de façon schématique la correction appliquée au signal analogique V- pour compenser les variations du courant d'obscurité ;
- la figure 7h montre l'intérêt de la compensation du courant d'obscurité sur les niveaux de gris obtenus dans le signal vidéo ;
- la figure 8 est un algorithme d'un programme informatique exécuté dans l'unité centrale 44 de la figure 2 ;
- la figure 9 est une vue schématique générale de la partie amovible de l'appareil de radiologie dentaire selon l'invention et qui est représentée de façon déployée ;
- la figure 10 est une vue de mise en situation de l'appareil de radiologie dentaire selon l'invention lors d'une prise d'image d'une dent ;
- la figure 11 est une vue éclatée du module électronique 32, de ses dispositifs d'anti-traction et des demi-coques métalliques de protection ;
- la figure 12 est une vue en perspective éclatée montrant l'implantation d'un corps métallique d'anti-traction et du câble associé dans le module électronique 32 ;
- la figure 13 est une vue schématique partielle d'une des extrémités du module électronique équipée d'un corps d'anti-traction et d'un câble associé ;
- la figure 14 est une vue en perspective éclatée des différents éléments logés à l'intérieur du boîtier 180 ;
- la figure 15 est une vue schématique partielle d'un des manchons d'extrémités représentés sur la figure 14 ;
- la figure 16 est une vue schématique en perspective des différentes pièces de la figure 14 une fois assemblées.

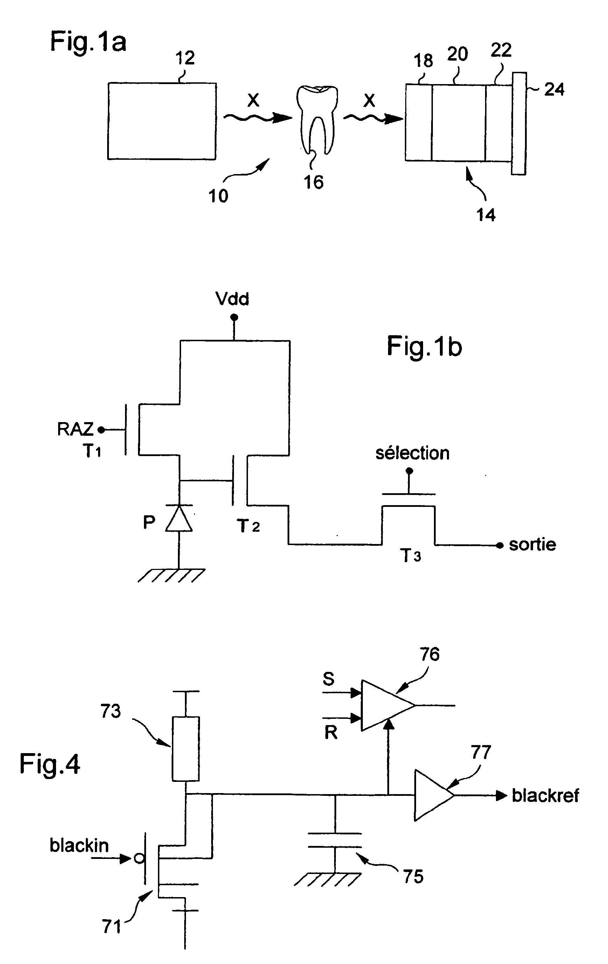

Comme représenté à la **figure 1a**, un appareil de radiologie dentaire à rayons X 10 comprend une source de rayonnement X 12 placée à l'extérieur de la bouche d'un patient et un capteur intra-buccal de rayonnement 14 disposé dans la bouche d'un patient, derrière une dent 16, et qui est apte à recevoir le rayonnement X qui irradie la dent.

Le capteur 14 comprend, suivant l'ordre de propagation du rayonnement, un scintillateur 18 qui convertit le rayonnement X ayant irradié la dent en rayonnement visible, une plaque de fibres optiques 20 qui, d'une part, comprend des particules métalliques destinées à absorber la partie du rayonnement X reçue par le scintillateur et non convertie en rayonnement visible et, d'autre part, conduit le rayonnement visible ainsi converti jusqu'à un détecteur 22. Ce détecteur est monté sur un substrat 24 en céramique et transforme le rayonnement visible issu des fibres optiques en un ou plusieurs signaux électriques analogiques.

Les différents composants du capteur 14 sont assemblés les uns avec les autres, par exemple, par collage.

Le scintillateur 18 est, par exemple, réalisé en oxysulfure de gadolinium.

Alternativement, il pourrait être réalisé en iodure de Césium, en cristaux de Lutécium ou en tout élément ayant la propriété de transformer le rayonnement X en rayonnement visible.

La plaque de fibres optiques 20 est, par exemple, commercialisée par la société SCHOTT sous la référence commerciale 47A ou par la société HAMAMATSU sous la référence commerciale XRS.

Le détecteur biCMOS 22 est un détecteur de type APS dit à pixels actifs (connu en terminologie anglosaxonne sous le terme "Active Pixel Sensor") utilisant la technologie de fabrication biCMOS, c'est-à-dire qui utilise à la fois des transistors NMOS et PMOS, par opposition aux détecteurs à pixels passifs. Pour plus de détails sur le détecteur biCMOS, on se reportera, par exemple, au document EP 0 858 111.

Un pixel actif intègre des moyens d'amplification de la charge électrique collectée sur l'élément du pixel qui est sensible à la lumière.

La charge électrique amplifiée qui est détenue par un pixel actif est appelée dans la suite de la description "valeur d'information" et est représentative de la quantité de lumière capturée par le pixel.

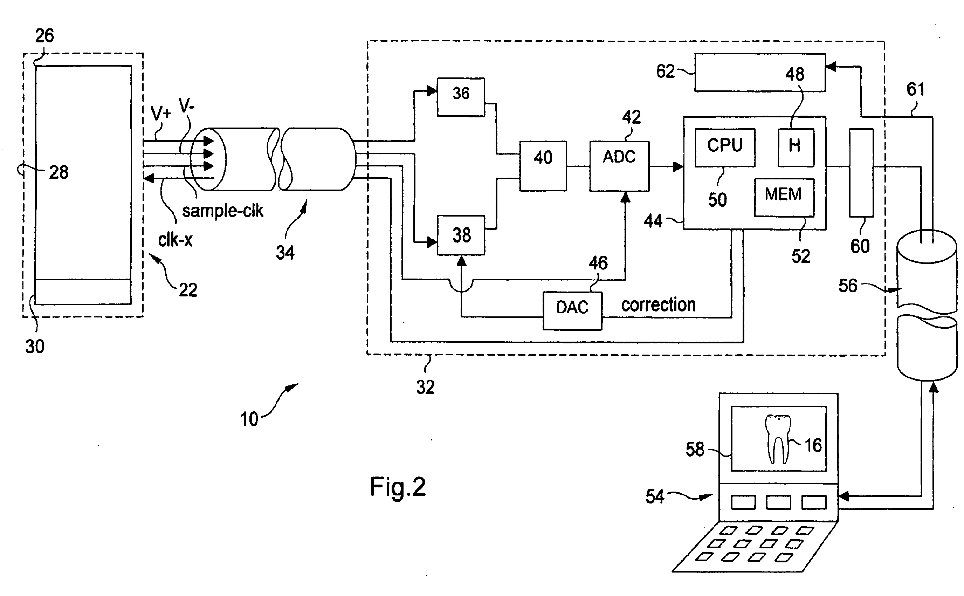

Le détecteur biCMOS 22 représenté sur la **figure 2** est une puce réalisée sur un substrat en silicium 28 suivant la technologie de fabrication biCMOS et qui comporte une matrice de pixels actifs 26 et un séquenceur 30 intégré sur le même substrat.

La structure des pixels est, par exemple, la structure connue illustrée à la **figure 1b** et mettant en oeuvre trois transistors T1, T2, T3 ainsi qu'une photodiode P.

De préférence, la photodiode a une forme adaptée à optimiser le coefficient de remplissage du pixel, tout en garantissant une canalisation suffisante des charges générées au niveau de la photodiode par l'impact des photons.

La forme de la photodiode doit en effet permettre de séparer de manière fiable les pixels les uns des autres afin d'éviter que la charge générée au niveau d'un pixel ne soit récupérée par un des pixels adjacents (diaphonie).

Il convient cependant de noter que la forme de la photodiode ne doit pas occuper une partie trop importante de la surface du pixel afin d'éviter de diminuer le coefficient de remplissage..

Le séquenceur 30 est apte à générer plusieurs signaux de commande pour la commande de la matrice.

Pour ce faire, le séquenceur reçoit un ou plusieurs signaux extérieurs au capteur et à partir duquel ou desquels sont générés les différents signaux de commande de la matrice.

L'appareil de radiologie 10 comprend un module électronique 32 distant du capteur 14 et donc du détecteur 22 de la figure 2 et qui est relié à ce capteur par une liaison filaire 34 qui est un câble.

Par exemple, le câble est de type multifilaire, multibrins, les signaux rapides (horloge, vidéo, ...) pouvant optionnellement faire l'objet de câbles coaxiaux et l'ensemble étant blindé par une tresse de masse.

Le ou les signaux extérieurs mentionnés ci-dessus sont générés dans le module électronique 32 et transmis par l'intermédiaire du câble 34 au détecteur 22 et, plus particulièrement au séquenceur 30 de ce dernier.

L'un des signaux extérieurs est, par exemple, un signal d'horloge unique Clk-x, par exemple doté d'une fréquence de 12 MHz et à partir duquel vont être générés par le séquenceur une pluralité de signaux internes nécessaires au fonctionnement de la matrice 26.

Il est particulièrement avantageux d'agencer le séquenceur dans le détecteur 22 et non dans le module électronique 32, puisque la seule transmission d'un nombre restreint de signaux dont le signal d'horloge unique provenant du module 32 suffit.

En effet, si le séquenceur était agencé dans le module électronique 32, il conviendrait alors de transmettre par le câble 34 un grand nombre de signaux destinés à la commande de la matrice, ce qui engendrerait un bruit supplémentaire, notamment en raison de la diaphonie créée entre les différents signaux transmis dans le câble.

Ainsi, l'agencement du séquenceur dans le détecteur 22, et notamment sur le même substrat que la matrice, permet d'améliorer le rapport signal sur bruit du détecteur.

On limite également par là même le nombre de fils passant dans le câble, ce qui permet d'utiliser un câble plus souple que si l'on devait faire transiter par ce dernier la pluralité de signaux de commande nécessaires au fonctionnement de la matrice.

Ceci est également avantageux dans la mesure où le câble 34 sort de la bouche du patient lorsque le capteur est placé à l'intérieur, derrière une dent, et qu'il est donc nécessaire de disposer d'un câble aussi peu rigide que possible pour minimiser la gêne occasionnée au patient.

On notera que l'intégration du séquenceur sur le même substrat que celui de la matrice permet de réduire l'encombrement général du détecteur, et donc du capteur, ce qui est primordial pour un capteur intra-buccal.

En effet, un séquenceur réalisé en tant que composant à part entière distinct de la puce du détecteur conduirait inévitablement à un capteur plus volumineux et donc plus gênant pour le patient.

La réduction du nombre de signaux à générer par le séquenceur tend à rendre celui-ci moins volumineux.

On notera que le module électronique 32 est placé à une distance du capteur d'au moins 50 cm pour ne pas gêner le patient ni même le dentiste et à une distance maximale d'environ 2 m du capteur pour des raisons qui seront développées ultérieurement.

Comme on le verra également plus loin, un ou plusieurs signaux de sortie analogiques sont élaborés dans le capteur et transmis par l'intermédiaire du câble 34 au module électronique 32 dans lequel ils subissent plusieurs traitements.

En particulier, le module 32 comporte deux unités d'amplification 36 et 38 qui amplifient chacune l'un des signaux de sortie analogiques qu'elle reçoit en entrée et une unité 40 de filtrage des deux signaux précités.

On pourrait également, de façon optionnelle, prévoir que l'unité 40 élabore un unique signal analogique de sortie à partir des deux signaux de sortie, en plus de la fonction de filtrage.

Les signaux de sortie analogiques filtrés sont ensuite réunis en un unique signal analogique qui est converti en un signal numérique dans un convertisseur analogique numérique 42 et transmis à une unité centrale de traitement 44.

L'unité centrale de traitement 44 comporte plus particulièrement une horloge 48, un microprocesseur 50 et une unité de mémorisation 52.

Cette unité de traitement 44 réalise entre autres, mais non exclusivement, des opérations de mise du détecteur dans le mode opératoire, par opposition aux périodes de veille, aiguille le signal numérisé vers l'interface de sortie 60 décrite ci-après, gère une unité de mémorisation 52 destinée, par exemple, à contenir la liste des éléments défectueux de la matrice 26 du détecteur 22 ou le numéro de série du détecteur, recherche sur le signal vidéo numérisé les informations caractéristiques de la présence ou non de rayonnement X et gère l'organe d'activation 100 décrit ultérieurement.

On notera également qu'une correction est appliquée à l'un des signaux analogiques amplifiés par l'unité 38 par l'intermédiaire d'un signal numérique de correction qui provient de l'unité centrale de traitement 44, est converti en un signal analogique de correction par un convertisseur numérique analogique 46 et est appliqué ensuite à l'unité 38.

L'unité 38 exerce la fonction d'un amplificateur soustracteur.

L'appareil de radiologie 10 comporte également une unité de traitement et de visualisation 54 distante du module électronique 32 et reliée à ce dernier par l'intermédiaire d'une liaison filaire 56 qui est, par exemple, un câble.

L'unité de traitement et de visualisation 54 est, par exemple, un ordinateur qui va recevoir les signaux de sortie du capteur 14, une fois numérisés et traités dans le module électronique 32, afin d'effectuer sur ces derniers un traitement d'image approprié et connu de l'homme de l'art afin de pouvoir visualiser sur l'écran 58 l'image de la dent 16 de la figure 1.

Une interface 60 conforme à la norme USB2.0 est prévue en sortie de l'unité 44, ainsi qu'un bus série USB2.0 et une interface correspondante dans l'ordinateur distant 46 (non représentée), pour que les signaux délivrés par le module 32 soient transmis à l'ordinateur distant 54 avec un haut débit, par exemple de l'ordre de 480 Mbits/s.

L'utilisation d'une telle interface permet ainsi de transmettre rapidement à l'ordinateur les données qui sont fournies par le détecteur CMOS 22 et traitées par le module 32.

L'utilisation de cette interface convient particulièrement bien à l'utilisation d'un signal d'horloge de fréquence élevée, par exemple à 12 MHz, pour l'échantillonnage des données recueillies par la matrice du détecteur biCMOS 22, ceci sans avoir à utiliser une mémoire tampon pour le stockage des données avant leur transmission à l'ordinateur.

En effet, cette fréquence de signal d'horloge représente un bon compromis entre, d'une part, une fréquence trop basse pour échantillonner la matrice, ce qui provoquerait une augmentation du courant d'obscurité entre le début et la fin de l'échantillonnage de la matrice et, d'autre part, une fréquence trop élevée qui générerait un bruit de lecture supplémentaire perturbant le signal de sortie du détecteur.

En effet, compte tenu des contraintes liées à l'évolution du courant d'obscurité dans le détecteur au cours du temps, il faut échantillonner les pixels de la matrice à une fréquence relativement élevée qui s'avère incompatible avec le débit de transmission autorisé par la norme USB1.

Il convient de noter que le bus USB2 du câble 56 véhicule les signaux d'image numérisés qui vont être transmis à l'unité 54 et un signal de tension unique 61 qui va servir au fonctionnement du détecteur CMOS après nettoyage dans un circuit 62 du module électronique 32.

Le détecteur biCMOS fonctionne en effet à partir d'une seule tension d'alimentation, par exemple, en 5 volts et de niveaux TTL de commande, contrairement à un détecteur à transfert de charge (CCD) pour lequel quatre ou cinq niveaux de tension différents doivent être générés ainsi que de nombreuses horloges.

Il convient de noter que la génération de différents niveaux de tension entraîne l'apparition de bruits supplémentaires qui perturbent le bon fonctionnement du capteur et que ce phénomène est donc évité dans l'appareil selon l'invention.

Le circuit 62 est en quelque sorte un convertisseur continu continu qui effectue un nettoyage de la tension d'alimentation fournie par le bus USB2 et qui permet également de s'affranchir des variations de la tension de 5 volts de ce bus en la stabilisant.

Il est à noter que les tensions continues générées par le convertisseur 62 peuvent être permanentes, par exemple à l'usage de l'unité centrale de traitement 44, ou commutées, par exemple à l'usage du détecteur 22, celui-ci n'étant mis sous tension que pendant les périodes d'utilisation en bouche. Ces commutations sont réalisées par l'unité centrale de traitement 44.

On notera que la vitesse de transmission des données sur la liaison filaire 56 doit être au moins égale à celle prévue par la norme USB2.0 afin de pouvoir vider rapidement la matrice 26 sans avoir recours à une mémoire tampon.

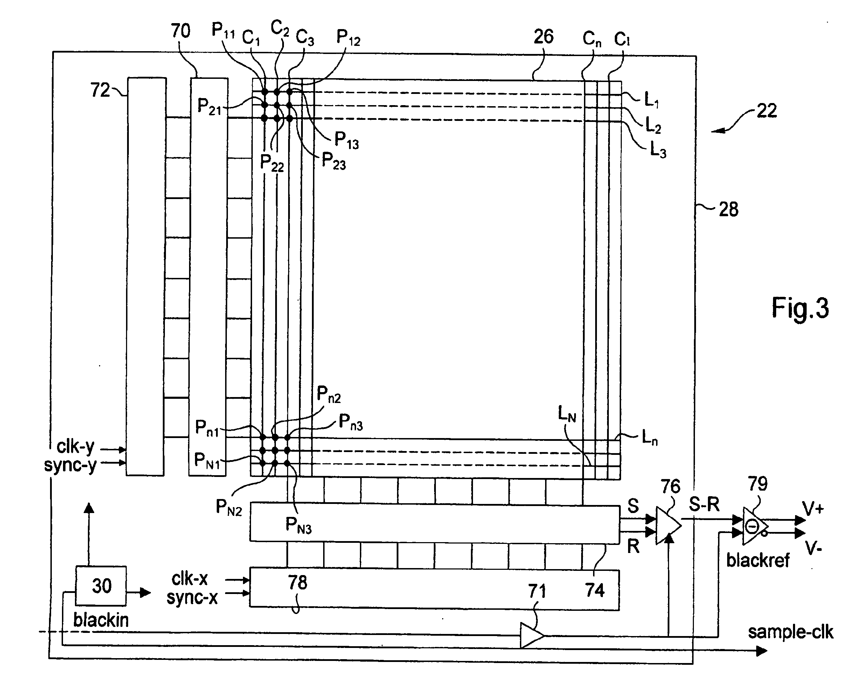

La **figure 3** représente de façon plus détaillée l'agencement de la matrice à pixels actifs 26 et le séquenceur 30 sur le substrat commun 28 de la puce du détecteur.

La matrice 26 est reliée à un circuit 70 de pilotage de la sélection et de la remise à zéro des lignes L₁, L₂, L₃, ... Lₙ, ..., L_{N} de la matrice.

Un circuit 72 de registres à décalage commandé par des signaux d'horloge et de synchronisation permet de contrôler le circuit 70 pour les opérations successives de sélection d'une ligne et de sa remise à zéro.

La matrice est également connectée à un circuit 74 d'amplification des colonnes C₁, C₂, C₃, ..., Cₙ, ..., Cₗ de la matrice et qui effectue un multiplexage sur un circuit d'amplification de sortie 76 des données d'image capturées par ladite matrice.

Un circuit 78 de registres à décalage des colonnes de la matrice est prévu pour contrôler le fonctionnement du circuit 74 et, notamment, piloter la lecture des pixels de chaque ligne de la matrice.

Comme annoncé plus haut, le détecteur reçoit plusieurs signaux du module électronique 32 et, notamment, les signaux Clk-x, Clk-y et Sync-y dont dérivent tous les signaux internes de la matrice.

Le signal Clk-x est un signal d'horloge, par exemple, à 12 MHz qui sert à piloter les registres à décalage des colonnes et constitue le principal signal d'horloge à partir duquel le séquenceur 30 génère tous les signaux, et notamment les signaux de commande de la matrice.

Le signal de commande Sync-y sert à initialiser la lecture de la matrice.

Le signal Clk-y est le signal de commande des registres à décalage des lignes commandant le circuit 72.

La lecture des données d'image capturées par la matrice s'effectue de la façon suivante.

Le signal Sync-y, appliqué en même temps que le signal Clk-y, initialise les registres à décalage des lignes de la matrice et provoque la sélection de la première ligne de la matrice.

Un premier signal de sélection de la première ligne de la matrice, qui est généré par le séquenceur 30 est déclenché, par exemple, sur un front montant du signal d'horloge Clk-y et commande la sélection de la première ligne de la matrice.

L'application du signal Clk-x va provoquer l'apparition du signal vidéo (signal de sortie) en sortie du détecteur.

L'application du seul signal Clk-y va provoquer la sélection de la ligne suivante de la matrice.

On procède ainsi jusqu'à ce que toutes les lignes aient été lues, puis on applique de nouveau la combinaison de signaux Sync-y et Clk-y pour sélectionner à nouveau la première ligne de la matrice.

Pour la ligne sélectionnée ainsi considérée, les différentes valeurs d'information détenues par chacun des pixels de la ligne ayant été exposés à un rayonnement vont subir une première étape d'échantillonnage, également appelée étape de lecture, selon une cadence imposée par le signal d'horloge Clk-x.

Chaque valeur d'information d'illumination du pixel est stockée dans le circuit d'amplification des colonnes 74.

Après une première étape d'échantillonnage des pixels de la première ligne, un signal Reset est généré par le séquenceur afin de remettre à zéro tous les pixels de la ligne considérée.

Cette étape de remise à zéro a pour effet d'initialiser tous les pixels de la ligne sélectionnée à une valeur d'information de référence propre à chaque pixel

Il s'agit d'une valeur d'information correspondant à un pixel non illuminé, également appelée valeur d'information d'obscurité.

Après initialisation des pixels de la ligne concernée, on procède à une deuxième étape d'échantillonnage ou de lecture des valeurs d'information de référence détenues par chaque pixel, au rythme imposé par le signal d'horloge Clk-x qui sélectionne les différentes colonnes de la matrice.

Les valeurs d'information de référence échantillonnées sont stockées dans le circuit d'amplification de colonnes 74 où elles rejoignent les valeurs d'information d'illumination déjà stockées et provenant de la première étape d'échantillonnage.

Les valeurs d'information des deux types sont alors multiplexées sur deux bus respectifs, l'un véhiculant un signal de lecture S (valeurs d'information d'illumination) et l'autre véhiculant un signal de réinitialisation R (valeurs d'information de référence).

Les données provenant de ces deux bus sont multiplexées sur le circuit d'amplification de sortie 76 selon une cadence imposée par le signal d'horloge Clk-x qui active les registres à décalage des colonnes.

Un signal de sortie S-R est formé par le circuit d'amplification de sortie 76 à partir de la différence entre le signal de lecture S et le signal de réinitialisation R et d'un signal de référence qui est représentatif des dérives électroniques intrinsèques du détecteur.

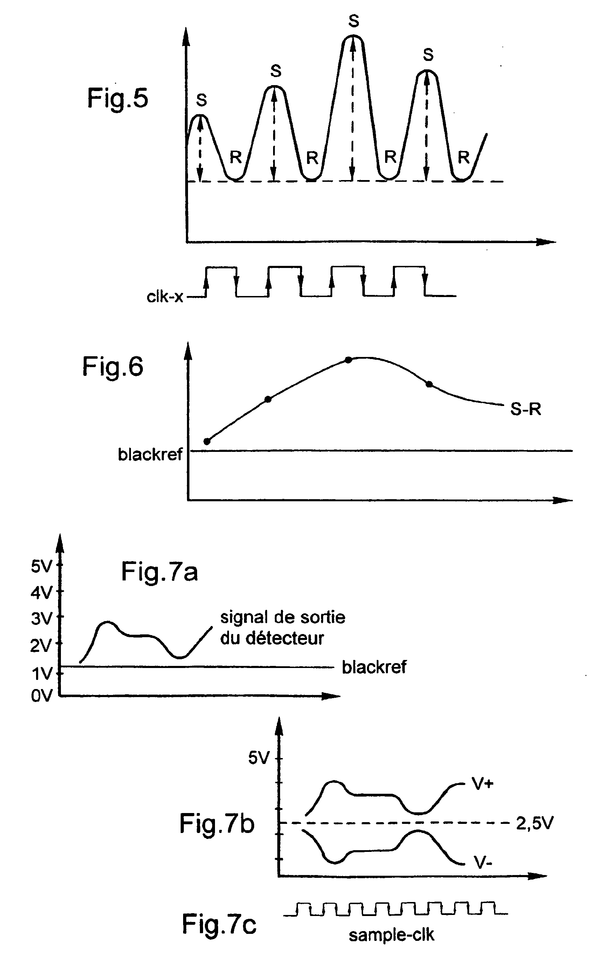

Ce signal de référence provient d'un signal de référence d'entrée "blackin" qui est une tension continue provenant du module 32 et appliquée en entrée du détecteur. Le signal de référence blackref est généré comme représenté sur la **figure 4.**

Le circuit de la figure 4 est représentatif de la manière dont le bruit est généré dans le détecteur et le signal blackin appliqué en entrée de ce circuit subit des dérives comme s'il passait dans la matrice.

Ce circuit comprend à cet effet un transistor 71 qui sert de mémoire tampon au signal d'entrée blackin et qui est également représenté schématiquement sur la figure 3.

Une résistance 73 et une capacité 75 dont les valeurs sont choisies de manière appropriée compte tenu du détecteur sont représentatives des éléments intrinsèques au détecteur.

Un amplificateur 77 amplifie le signal obtenu après qu'il ait subi les déformations provoquées par les éléments 71, 73 et 75 et le signal amplifié blackref est ainsi délivré en sortie du détecteur 22.

Le signal obtenu avant passage dans l'amplificateur 77 ("signal de référence non amplifié") est fourni au circuit d'amplification de sortie 76 où, avec le signal d'horloge Clk-x, on sélectionne sur l'un des fronts montant ou descendant du signal Clk-x la valeur la plus forte du signal S-R et, sur l'autre front, la valeur fournie par le signal de référence non amplifié comme illustré sur la figure 5.

On obtient ainsi un signal S-R dont les plus hautes valeurs sont celles du signal de lecture de la matrice et les plus basses valeurs sont celles du signal de référence non amplifié, ces dernières valeurs pouvant varier au cours du temps.

Le signal S-R obtenu (figure 6) est représentatif des données d'image de la dent qui ont été capturées par les pixels du détecteur.

La figure 7a représente l'allure générale du signal R-S de sortie du détecteur (figure 3) et du signal de référence de sortie blackref.

Comme représenté sur la figure 3 , le signal électrique analogique de sortie S-R issu du détecteur est transformé par un générateur de signaux 79 en deux signaux électriques analogiques différentiels V+ et V-.

Le générateur 79 est un amplificateur à deux sorties qui soustrait le signal blackref au signal S-R et délivre les signaux différentiels V+ et V- dont l'allure est représentée sur la figure 7b.

La superposition de la différence de signaux S-R au signal de référence de sortie blackref permet de s'affranchir des dérives dues à la construction du détecteur 22 qui sont constantes dans le temps et qui varient d'un capteur à l'autre.

En transmettant sous forme différentielle le signal d'image issu du capteur, on s'affranchit des différentes perturbations auxquelles il peut être soumis.

En effet, comme chacun des deux signaux V+ et V- est soumis aux mêmes perturbations, on peut alors, en reconstituant un signal unique s'affranchir des perturbations constatées sur chacun des signaux V+ et V-, ce qui ne serait pas le cas si l'on ne transmettait qu'un seul signal d'image.

Par ailleurs, la transmission d'un signal d'image différentiel permet également d'améliorer indirectement le rapport signal sur bruit du capteur.

Un signal d'échantillonnage Sample-clk est généré par le séquenceur (figure 3) et est, par construction, parfaitement synchrone avec le signal de sortie S-R dans la mesure où le séquenceur a une connaissance précise de l'instant où les données vont être présentées sur la sortie du détecteur. Le signal d'échantillonnage est élaboré par un déphasage adapté du signal d'horloge Clk-x et est représenté à la figure 7c.

Ce signal d'échantillonnage qui est en phase avec le signal de sortie S-R, et donc avec les signaux différentiels V+ et V-, va être transmis simultanément avec ces derniers par le câble 34 à destination du module électronique 32.

La transmission simultanée des signaux en phase va permettre, à l'intérieur du module électronique 32, d'effectuer dans le circuit de conversion analogique numérique 42 la conversion des signaux analogiques différentiels à la fréquence imposée par le signal d'échantillonnage.

En transmettant les signaux simultanément, cela permet de s'affranchir d'erreurs de phase survenant dans le câble dans la mesure où une erreur de phase affectant à la fois les signaux analogiques différentiels et le signal d'échantillonnage pourra être compensée.

On s'affranchit par là même d'éventuelles autres perturbations affectant les signaux lors de leur transmission.

Comme représenté sur la figure 2, les signaux analogiques de sortie différentiels V+ et V- sont respectivement amplifiés dans des circuits d'amplification 36 et 38 avant d'être transmis au circuit de filtrage 40.

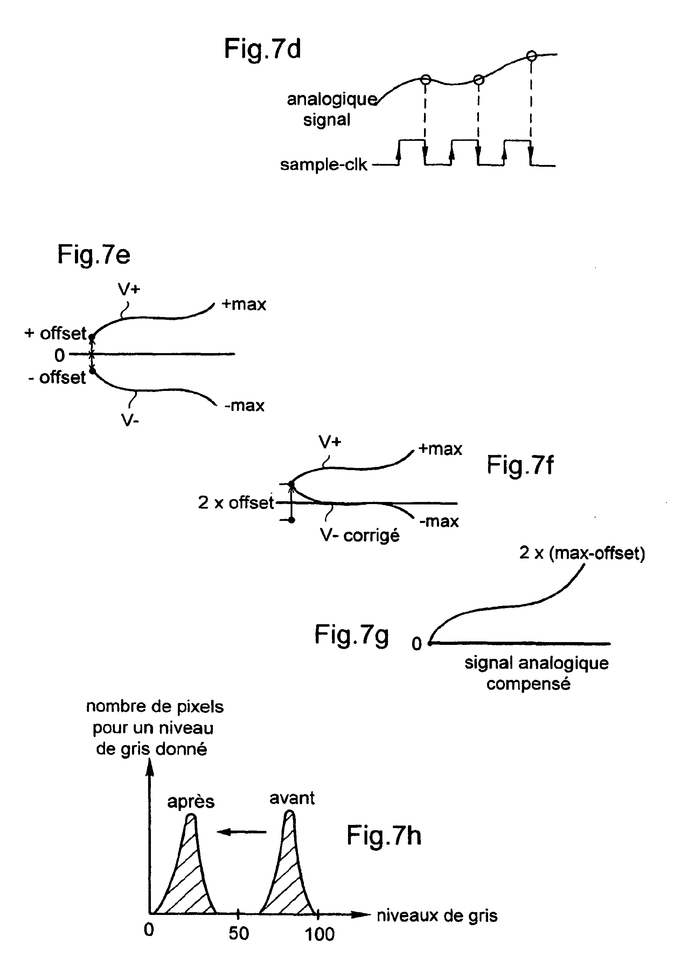

Les signaux ainsi filtrés sont transmis au convertisseur analogique numérique 42 où ils sont réunis en un signal unique par sommation et ce signal est numérisé à l'aide du signal d'échantillonnage Sample-clk (par exemple sur le front descendant du signal d'échantillonnage, comme représenté à la figure 7d) avant de parvenir à l'unité centrale de traitement 44.

Afin de s'affranchir des variations temporelles du courant d'obscurité qui est défini comme étant le courant électrique recueilli en sortie du détecteur lorsque ce dernier n'est exposé à aucun rayonnement, on procède à la génération d'un signal de correction entre deux phases d'exposition de la matrice à un rayonnement qui sont, par exemple, successives.

On notera que la génération d'un tel signal peut être régulière ou non dans le temps.

On effectue ainsi en dehors d'une prise d'image d'une dent par le capteur un échantillonnage des valeurs d'information détenues par les pixels de la matrice lorsque celle-ci n'est exposée à aucun rayonnement. Le signal de lecture de la matrice qui est généré est un signal de correction.

De façon similaire à ce qui a été décrit précédemment pour les signaux de sortie V+ et V-, le signal de lecture en l'absence de rayonnement est transmis sous forme différentielle dans le câble 34 puis amplifié dans les circuits d'amplification 36 et 38 avant d'être reconstitué en un signal unique par le circuit 40 et numérisé dans le convertisseur 42.

Ce signal numérisé est introduit dans l'unité centrale 44 dans laquelle le microprocesseur calcule une valeur moyenne de ce signal numérisé qui n'est pas nulle, contrairement à ce que l'on devrait obtenir en l'absence de rayonnement. On injecte cette valeur moyenne dans le convertisseur numérique analogique 46 pour la transformer en un signal analogique de correction appliqué à l'un, 38, des circuits d'amplification qui est un circuit soustracteur.

L'application de ce signal de correction à l'un des signaux analogiques de sortie, en l'espèce le signal V-, permet de décaler l'amplitude de ce signal en ramenant ce signal vers le haut comme représenté sur les figures 7e et 7f.

La figure 7e représente de façon très schématique l'allure des signaux de sortie différentiels V+ et V- qui sont chacun décalés par rapport au niveau zéro d'une même valeur (offset).

En mesurant une valeur moyenne (2 x offset) sur le signal de correction numérisé et en l'appliquant, après conversion numérique analogique, au signal de sortie analogique V-, on décale ce dernier d'une valeur corrigée de 2 x offset, comme représenté sur la figure 7f, pour le ramener au niveau bas de l'autre signal de sortie analogique non corrigé V+.

Lorsque le signal V+ et le signal corrigé V- sont filtrés et réunis, on obtient alors le signal analogique compensé représenté à la figure 7g qui part de zéro jusqu'à une amplitude maximale de 2 x (max-offset), où la valeur "max" désigne l'amplitude maximale en valeur absolue de chacun des signaux V+ et V-.

Le signal analogique compensé est alors numérisé dans le circuit 42.

On s'affranchit ainsi des variations dues au courant d'obscurité dans la matrice.

La figure 7h, qui représente le nombre de pixels en fonction des niveaux de gris présents dans le signal vidéo, illustre ce phénomène de compensation en montrant l'allure de l'histogramme du signal vidéo avant et après l'application d'une correction.

On notera qu'il est préférable de convertir le signal analogique de correction plutôt que de l'appliquer directement à l'un des signaux analogiques de sortie, dès réception du signal de correction par le module électronique 32.

En effet, ceci nécessiterait de stocker le signal analogique de correction avec tous les risques de dérives et/ou de volatilité que cela comporte.

En effectuant cette correction de façon numérique, on évite ces problèmes et on intègre en plus le convertisseur analogique numérique dans la compensation, ce qui évite d'entacher le processus de numérisation par une dérive qui est propre au convertisseur.

Par ailleurs, on notera que l'on peut également appliquer un signal analogique de correction à l'autre signal analogique de sortie V+, voire aux deux signaux V+ et V-.

L'application d'une correction aux deux signaux symétrise le processus.

L'application d'une correction à l'un des signaux de sortie analogiques ou au signal analogique unique est adaptée en fonction de la loi de variation en fonction du temps du courant d'obscurité de la matrice et de la durée pendant laquelle cette matrice est mise en oeuvre.

En effet, connaissant ces deux paramètres, il est possible de prévoir à quel moment le courant d'obscurité est susceptible de varier le plus et donc d'envisager d'effectuer une correction pour compenser les dérives dues à ces variations.

Comme expliqué ci-dessus, lors de la lecture de la matrice et, plus particulièrement, après avoir effectué un premier échantillonnage des pixels d'une ligne sélectionnée de la matrice, un signal de remise à zéro est appliqué à la ligne concernée de la matrice par l'intermédiaire des circuits 70 et 72 (figure 3) pour réinitialiser les valeurs des pixels de la ligne à des valeurs d'information de référence (valeurs d'information d'obscurité).

Or, si le signal de remise à zéro varie d'une ligne à l'autre, on introduit une erreur dans les valeurs d'information détenues par les pixels de la ligne, ce qui se traduit par des décalages par rapport au zéro : la valeur d'information détenue par un pixel après sa réinitialisation n'est pas nulle comme elle devrait théoriquement l'être, mais présente un décalage par rapport au zéro et, de plus, ce décalage peut varier d'une ligne à l'autre.

L'introduction d'une erreur supplémentaire se traduit par une détérioration du rapport signal sur bruit fourni par le détecteur.

Plus particulièrement, avec de telles erreurs, on introduit un bruit de "peignage horizontal" dans le signal d'image produit sur l'écran 58 de l'ordinateur 54 (figure 2).

Pour remédier à ce problème, on prévoit d'abord de rendre optiquement inactif un nombre m de pixels de chacune des lignes de la matrice, les pixels optiquement inactifs des différentes lignes étant en nombre égal (par exemple égal à 3) et agencés dans les mêmes colonnes de la matrice.

On notera que les pixels rendus optiquement inactifs sont agencés dans les premières colonnes de la matrice mais pourraient tout aussi bien se trouver à un autre endroit, tel que dans les dernières colonnes de celle-ci.

On notera que le nombre m de pixels optiquement inactifs peut varier en fonction de la précision que l'on souhaite apporter lors de la compensation de la dérive introduite avec le signal de réinitialisation.

Le nombre m peut également varier en fonction de la taille de la matrice.

Dans l'exemple de réalisation illustré à la figure 3, les lignes L₁, L₂, ..., Lₙ, ..., L_{N} comportent respectivement chacune trois pixels "aveugles" P_{1.1,} P_{1.2}, P_{1.3}, P_{2.1}, P_{2.2}, P_{2.3}, ..., Pₙ₁, Pₙ₂, Pₙ₃, ...,P_{N1}, P_{N2} et P_{N3}.

Pour rendre un pixel optiquement inactif, on effectue, par exemple, une métallisation sur ces pixels ou bien une sérigraphie.

On effectue ensuite un traitement du signal de sortie du détecteur après numérisation par le circuit 42 (figure 2).

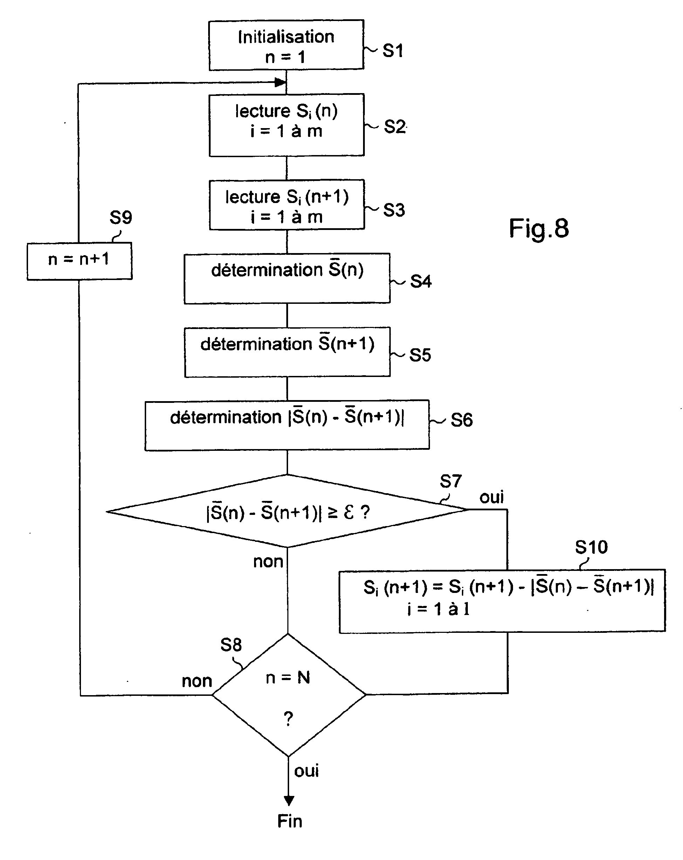

Le traitement est appliqué au signal par l'unité centrale de traitement 44 par l'intermédiaire du processeur 50 qui va exécuter une série d'instructions prévues dans l'algorithme de la figure 8 qui est stocké dans la mémoire 52.

Cet algorithme comporte une première étape S1 d'initialisation au cours de laquelle la variable n représentative des lignes de la matrice est initialisée à la valeur 1.

Au cours de l'étape suivante S2, on procède à la lecture des valeurs d'information Sᵢ(n) de la ligne n de la matrice pour les trois pixels aveugles P_{n1,} Pₙ₂, Pₙ₃.

Ces valeurs sont lues dans le signal de sortie numérisé.

Au cours de l'étape suivante S3, on procède à la lecture des valeurs d'information Sᵢ(n+1) provenant respectivement des trois pixels optiquement inactifs P_{n+1.1}, P_{n+1.2} et P_{n+1.3}, de la ligne suivante n+1 de la matrice.

Après avoir procédé à la lecture des valeurs d'information provenant des pixels optiquement inactifs de deux lignes consécutives n et n+1 de la matrice, on procède, au cours des étapes suivantes S4 et S5, à la détermination pour chacune des lignes n et n+1 d'une valeur moyenne d'information *S̅*(n), *S̅*(n+1) qui est obtenue à partir des valeurs d'information Sᵢ(n), Sᵢ(n+1) respectives de chaque ligne.

Plus le nombre m est élevé, meilleure sera la précision sur la détermination de la valeur moyenne.

La valeur moyenne d'information est, par exemple, obtenue en effectuant une moyenne arithmétique.

Au cours de l'étape S6, on procède à la détermination de la valeur absolue de la différence entre les deux valeurs moyennes précédemment déterminées.

L'étape suivante S7 est un test qui effectue une comparaison entre les valeurs moyennes d'information *S̅*(n) et *S̅*(n+1).

En théorie, on examine si les deux valeurs moyennes sont égales mais, en pratique, on compare le résultat obtenu à l'étape S6 avec une valeur seuil ε qui tient compte, par exemple, de l'ordre de grandeur des différences qu'il est techniquement possible de détecter entre les valeurs moyennes.

Lorsqu'aucune différence significative n'est constatée entre les valeurs moyennes d'information *S̅*(n) et *S̅*(n+1), alors l'étape suivante S8 est un test au cours duquel on vérifie si la variable représentative du nombre de lignes n est égale au nombre total N de lignes de la matrice.

Dans l'affirmative, il est mis fin à l'algorithme.

Au contraire, s'il reste des lignes de la matrice à traiter, alors l'étape S8 est suivie d'une étape S9 au cours laquelle on incrémente d'une unité la variable n et l'on effectue alors de nouveau les étapes qui viennent d'être décrites.

Lorsque le résultat du test pratiqué à l'étape S7 est positif, alors il est décidé de modifier, dans le signal de sortie numérique les valeurs d'information de tous les pixels de la ligne n+1 comme illustré par l'étape S10.

Au cours de cette étape, la valeur d'information provenant de chacun des 1 pixels de la ligne n+1 et qui est notée Sᵢ(n+1) est modifiée en affectant à celle-ci la valeur absolue de la différence déterminée à l'étape S6, afin de ramener la valeur moyenne d'information *S̅*(n+1) de la ligne n+1 à la valeur moyenne d'information *S̅*(n) de la ligne n.

L'étape S10 est ensuite suivie de l'étape S8 telle que décrite ci-dessus.

En procédant de cette façon pour chaque paire de lignes consécutives, on corrige chacune des lignes de la matrice, à l'exception de la première et on homogénéise donc l'image ligne à ligne afin de supprimer le bruit de "peignage horizontal" mentionné plus haut.

On notera qu'il est également possible d'effectuer une comparaison, non plus sur deux lignes consécutives de la matrice mais sur trois lignes consécutives.

Dans cette éventualité, on procède alors à la lecture de chacune des valeurs d'information provenant des m pixels optiquement inactifs d'un ensemble de trois lignes consécutives de la matrice et l'on détermine, pour chacune de ces lignes, une valeur moyenne d'information.

Après comparaison des valeurs moyennes d'information des trois lignes, en fonction du résultat de la comparaison, on décide alors on non de modifier dans le signal de sortie numérique les valeurs d'informations de tous les pixels de la deuxième ligne.

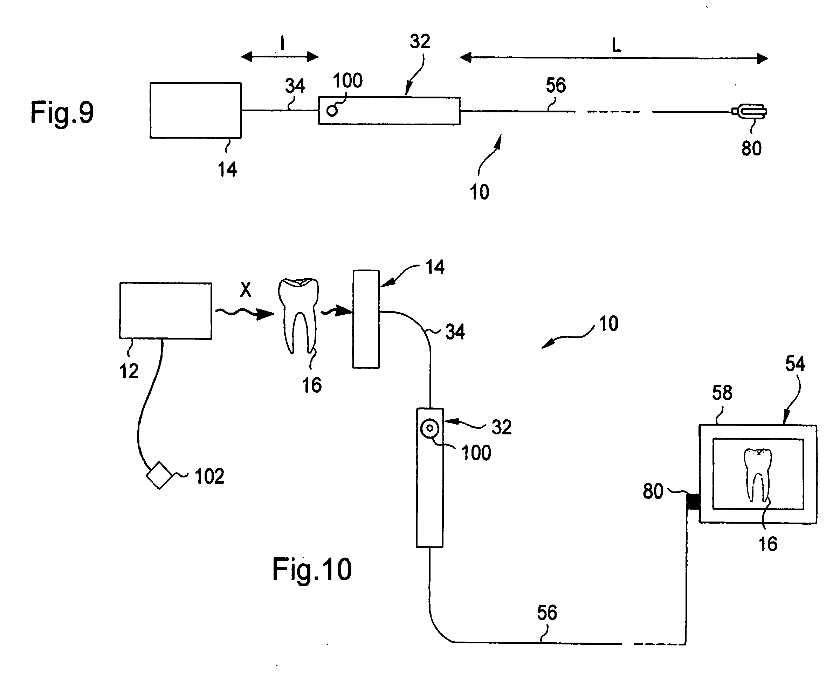

On a représenté sur **la figure 9** la réalisation physique d'un appareil de radiologie dentaire selon l'invention qui comporte le capteur intra-buccal 14, le module électronique 32 intégré dans un boîtier sur lequel on reviendra ultérieurement, les deux éléments étant reliés l'un à l'autre par un câble 34, et un connecteur 80 relié au module 32 par le câble 56.

Le connecteur 80 est destiné à coopérer avec un connecteur complémentaire de l'unité de traitement et de visualisation 54.

On notera que le câble 34 est relativement court dans la mesure où il véhicule des signaux analogiques qui risqueraient de s'atténuer trop fortement si le câble était trop long.

Cet agencement permet de tenir compte des contraintes spécifiques à la radiologie dentaire et notamment du fait que l'intensité du signal en sortie du capteur 14 est limitée par le fait que la dose de rayons X irradiant la ou les dents du patient est nécessairement maintenue aussi faible que possible afin de ne pas exposer ce dernier à de trop fortes de doses de rayonnement.

Pour cette raison, la longueur l du câble 34 est généralement inférieure à 2 mètres.

La longueur réduite I du câble 34 permet ainsi de ne pas dégrader le rapport signal sur bruit du capteur 14.

D'autre part, cette longueur I est généralement supérieure à 50 cm afin de ne pas occasionner une gêne supplémentaire en le plaçant trop près de la bouche du patient.

On notera par contre que la longueur L du câble 56 qui transmet des signaux numériques n'est, quant à elle, pas limitée et peut, par exemple, être égale à plusieurs mètres et, notamment, comprise entre 2 et 5 mètres.

Le module électronique 32 comporte un organe d'activation du détecteur 100 qui se présente, par exemple, sous la forme d'un bouton poussoir.

Cet organe 100 est particulièrement utile aux dentistes pour réveiller le détecteur du capteur 14 et le mettre en état de recevoir et traiter un rayonnement X ayant irradié une dent 16 du patient, dans la mesure où le module 32 est placé, lors de son utilisation, dans la sphère d'activité du dentiste.

Ceci signifie qu'il peut donc, sans avoir à se déplacer, prendre en main le module 32 et appuyer sur l'organe d'activation du détecteur 100.

Cet agencement permet au dentiste de ne pas se déplacer jusqu'à l'unité distante 54 pour cliquer sur une souris d'ordinateur avec ses gants d'intervention qui lui ont servi à placer le capteur 14 dans la bouche du patient et qui sont donc déjà contaminés, notamment par la salive du patient.

On notera que dans l'art antérieur, en cliquant sur la souris, on active le capteur intra-buccal par l'intermédiaire d'une interface logicielle.

On évite ainsi des risques de contamination croisée avec un autre patient dans la mesure où la souris ne fait généralement pas partie du matériel qui est désinfecté après utilisation.

Après activation du capteur à l'aide de l'organe d'activation 100, le dentiste allume le générateur de rayons X 12 au moyen d'un organe de déclenchement 102 tel qu'une poire électrique (**figure 10**) qu'il a à portée de main.

En outre, comme représenté sur la figure 10, lors de l'utilisation de l'appareil de radiologie dentaire selon l'invention, le module électronique encapsulé est suspendu au capteur 14 lorsque celui-ci est placé dans la bouche d'un patient, ce qui lui permet d'être à portée de main du dentiste lorsque celui-ci porte ses gants et qu'il vient d'installer le capteur dans la bouche du patient.

Il est en effet possible de suspendre le module électronique encapsulé dans son boîtier au bout du câble 34 le reliant au capteur dans la mesure où il est conçu pour que son poids et ses dimensions permettent cette utilisation.

En effet, un poids trop élevé risquerait de tirer sur le câble 34, et donc d'exercer une traction sur le capteur 14, ce qui serait ressenti par le patient et occasionnerait une gêne supplémentaire.

Par ailleurs, les dimensions raisonnables du module encapsulé sont adaptées à la taille d'une main d'adulte et ne provoquent ainsi aucune gêne supplémentaire liée à l'encombrement, ni pour le dentiste, ni pour le patient.

On notera également que l'organe d'activation 100 du module électronique 32 ou plusieurs organes d'activation peuvent être prévus pour réaliser d'autres fonctions.

Dans la mesure où un seul organe d'activation 100 est prévu, ces autres fonctions peuvent être mises en oeuvre par des appuis successifs sur ce dernier.

Ainsi, lorsqu'une image est en train d'être prise par le capteur ou bien après la prise d'image, il est utile de pouvoir fournir au logiciel de traitement d'image installé dans l'unité distante 54 l'information sur le sens de l'orientation de l'image, par exemple, suivant que l'on prenne une image d'une dent du haut, que l'on place le capteur horizontalement pour prendre une image d'une occlusion, ou bien que l'on prenne une image d'une dent du bas.

Ainsi, par plusieurs appuis successifs sur l'organe 100, on va repérer dans quelle position le capteur a été placé par le dentiste.

A titre d'exemple, un appui sur l'organe 100 allume le capteur et indique qu'il s'agit d'une prise d'image d'une dent du bas, deux appuis brefs allument le capteur et indiquent qu'il s'agit d'une prise d'image d'une occlusion avec le capteur placé horizontalement, tandis que trois appuis successifs permettent de rallumer le capteur et d'indiquer que l'image est retournée dans la mesure où on a pris une dent du haut verticalement.

D'autres fonctions telles que, par exemple, le réglage de la luminosité ou du contraste de l'image, peuvent également être commandées par l'activation de l'organe 100.

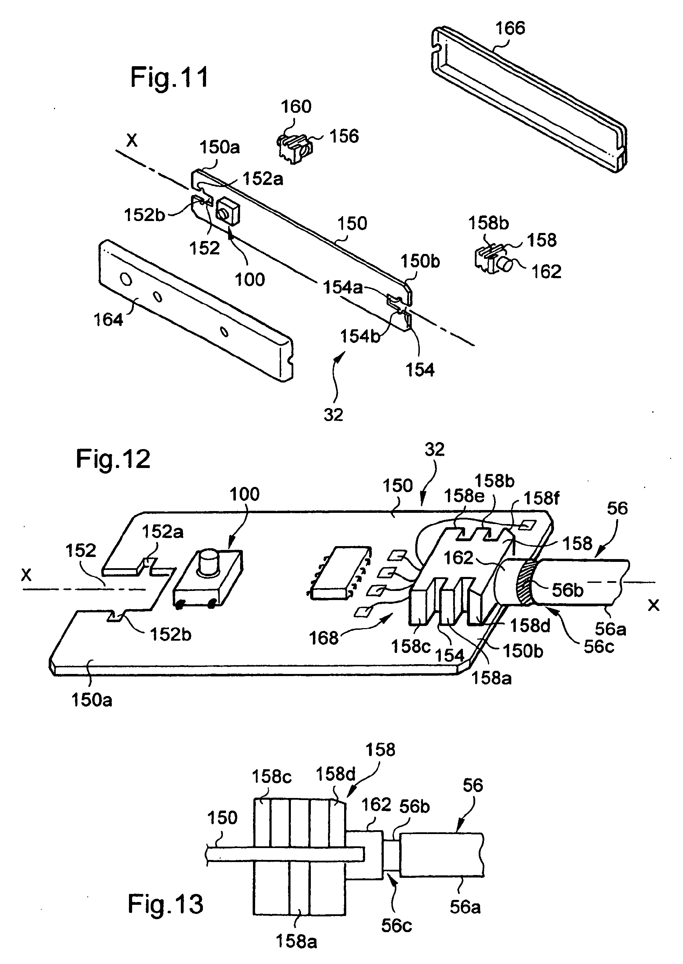

Comme représenté sur la **figure 11,** le module électronique 32 se présente sous la forme d'un circuit imprimé 150 de forme générale allongée le long d'un axe longitudinal X.

Le circuit imprimé 150 a ici une forme générale rectangulaire et comporte à chacune de ses deux extrémités opposées 150a, 150b qui sont disposées longitudinalement suivant l'axe X, une découpe axiale 152, 154 de forme, par exemple, rectangulaire et ouverte vers l'extérieur du circuit.

Les découpes axiales 152, 154 sont destinées à loger un corps métallique d'anti-traction 156, 158 (figure 11) suivant l'axe longitudinal du circuit.

Le module électronique 32 est équipé de deux dispositifs anti-traction qui sont chacun aptes à coopérer avec une des extrémités de l'un des câbles 34 et 56, de manière à empêcher le retrait du câble correspondant du module électronique et du boîtier sous l'action d'une traction exercée sur ledit câble.

Chaque câble comporte une gaine (gaine 56a pour le câble 56 sur la **figure 12**) qui est coaxiale à un faisceau de fils électriques (faisceau 56b du câble 56).

Au droit de chaque extrémité de chaque câble qui s'insère dans le boîtier comme on le verra ultérieurement, la partie du faisceau de fils correspondant 56b, au droit de l'extrémité 56c du câble 56, est solidarisée au corps métallique d'anti-traction 158.

On notera que tout ce qui est décrit pour le câble 56 et le corps métallique d'anti-traction 158 est valable pour le câble 34 et le corps d'anti-traction 156.

Chacun des faisceaux de fils (tresse du câble) est solidarisé au corps métallique d'anti-traction correspondant par l'intermédiaire d'un fût cylindrique, noté 160 pour le corps 156 et 162 pour le corps 158, qui fait lui-même partie intégrante dudit corps.

Plus particulièrement, le câble, par exemple le câble 56 de la figure 12, est dénudé à son extrémité 56c afin de pouvoir introduire le faisceau de fils 56b à l'intérieur du fût 162 et, ensuite, effectuer un soudage de la partie extrême du faisceau de fils dans le fût.

Ainsi, les faisceaux de fils sont solidarisés indirectement au corps métallique d'anti-traction par l'intermédiaire du fût correspondant, mais on pourrait très bien envisager de souder directement les faisceaux de fils sur le corps lui-même.

Comme représenté sur la figure 11, les corps métalliques d'anti-traction 156 et 158 sont introduits dans les fentes longitudinales ou découpes correspondantes 152 et 154 perpendiculairement à l'axe longitudinal X du circuit 150.

Ceci s'explique par le fait que chaque corps métallique d'anti-traction est pourvu d'éléments d'emboîtement agencés sur des faces opposées parallèles à la direction de la partie d'extrémité du faisceau de fils qui est solidarisée au corps (figure 12), direction qui est confondue avec l'axe longitudinal X du circuit 150 lorsque le corps métallique d'anti-traction est en position sur ledit circuit.

Plus particulièrement, les éléments d'emboîtement 158a et 158b du corps d'anti-traction 158 se présentent sous la forme de saillies présentes sur toute la hauteur du corps (**figure 13**) et qui coopèrent avec des éléments d'emboîtement complémentaires aménagés respectivement sur les bords opposés longitudinaux de la découpe correspondante.

Ces éléments d'emboîtement complémentaires se présentent sous la forme d'encoches transversales qui sont représentées sur la figure 11 et , de façon plus visible, sur la figure 12 pour la découpe 152.

Ces encoches 152a et 152b coopèrent avec les éléments d'emboîtement saillants du corps correspondant et constituent un montage de type tenon-mortaise.

Les encoches 154a et 154b de la découpe 154 par laquelle est introduit le corps métallique d'anti-traction 158 sont représentés sur la figure 11.

On notera que les éléments d'emboîtement du corps d'anti-traction et les éléments complémentaires aménagés dans la découpe correspondante constituent des éléments de retenue axiale du corps d'anti-traction, empêchant tout retrait axial du corps par rapport au circuit imprimé 150.

Ces éléments d'emboîtement facilitent également la mise en place du corps métallique d'anti-traction par un guidage approprié selon une direction transversale (figure 11).

La présence des dispositifs d'anti-traction permet de se passer de connecteurs électriques, ce qui est particulièrement avantageux.

En effet, l'utilisation de connecteurs électriques risquerait de conduire à des déconnexions intempestives, ce qui n'est pas souhaitable lorsque le patient vient d'être exposé à une dose de rayonnement X et qu'il est alors nécessaire de l'exposer une nouvelle fois à ces rayonnements après avoir rétabli la connexion électrique au niveau du module électronique.

En outre, la présence de connecteurs électriques équipés de systèmes de verrouillage sophistiqués est peu souhaitable dans la mesure où cela complexifierait l'appareil et ajouterait du poids et du volume au module électronique encapsulé dans son boîtier, ce que l'on cherche tout particulièrement à éviter.

Par ailleurs, la présence d'un connecteur électrique à proximité du patient peut s'avérer problématique en cas de déconnexion intempestive ou de faux contact pour des raisons de sécurité.

D'autre part, le boîtier 180 qui renferme le module électronique doit être désinfecté après chaque intervention du dentiste. Or, la présence d'un connecteur électrique mâle et d'un connecteur électrique femelle nécessite des réceptacles pour les branches des connecteurs et de tels aménagements sont des endroits qui ne pourront jamais être stérilisés, du moins d'une façon satisfaisante.

En outre, des problèmes d'étanchéité risquent de survenir lors de la stérilisation, ce qui n'est pas acceptable.

Chaque corps d'anti-traction est également pourvu sur ses deux faces opposées parallèles qui portent les éléments d'emboîtement, de deux éléments de butée transversale agencés de part et d'autre d'un des éléments d'emboîtement et qui sont référencés 158c, 158d, pour ceux encadrant l'élément d'emboîtement 158a, et 158e et 158f, pour ceux encadrant l'élément d'emboîtement 158b (figures 12 et 13).

Ces éléments de butée transversale sont moins longs que les éléments d'emboîtement afin que, lors de l'introduction des éléments d'emboîtement de chaque corps d'anti-traction à l'intérieur des évidements correspondants de la découpe, les éléments de butée transversale viennent en butée contre les bords longitudinaux de la découpe afin d'immobiliser le corps d'anti-traction dans cette position (figure 13).

On notera que les éléments d'emboîtement aménagés, d'une part, sur le corps métallique d'anti-traction et, d'autre part, sur les bords de la découpe correspondante peuvent être inversés, en ce sens que le corps peut être pourvu de rainures longitudinales et la découpe d'ergots correspondants.

D'autres éléments d'emboîtement peuvent être envisagés (montage en queue d'arronde, plusieurs encoches sur chaque bord de la découpe, encoches de formes différentes ...).

On notera que la carte 150 comporte l'organe d'activation 100 se présentant sous la forme d'un bouton poussoir et qui permet d'activer le détecteur lorsqu'une image d'une dent doit être prise.

Comme représenté sur la figure 11, deux demi-coques métalliques 164 et 166 sont agencées de part et d'autre du circuit imprimé 150 et sont assemblées l'une avec l'autre, par l'intermédiaire d'un cordon de soudure, de manière à emprisonner ledit circuit imprimé.

Ces coques métalliques sont assemblées après que les corps métalliques d'anti-traction soient solidarisés aux câbles 34 et 56, que ces corps soient positionnés dans les découpes correspondantes du circuit 150 et que les connexions électriques 168 avec la carte (figure 12) soient mises en place.

On notera que les corps métalliques d'anti-traction servent également d'entretoises pour permettre la fixation des demi-coques métalliques.

Ces dernières remplissent plusieurs fonctions :
- une première fonction de ces demi-coques métalliques est d'assurer une continuité électrique entre les masses des deux câbles 34 et 56 ;
- une deuxième fonction est de réaliser ainsi une cage de Faraday à des fins de compatibilité électromagnétique ;
- une troisième fonction de ces demi-coques est de protéger mécaniquement le module électronique 32.

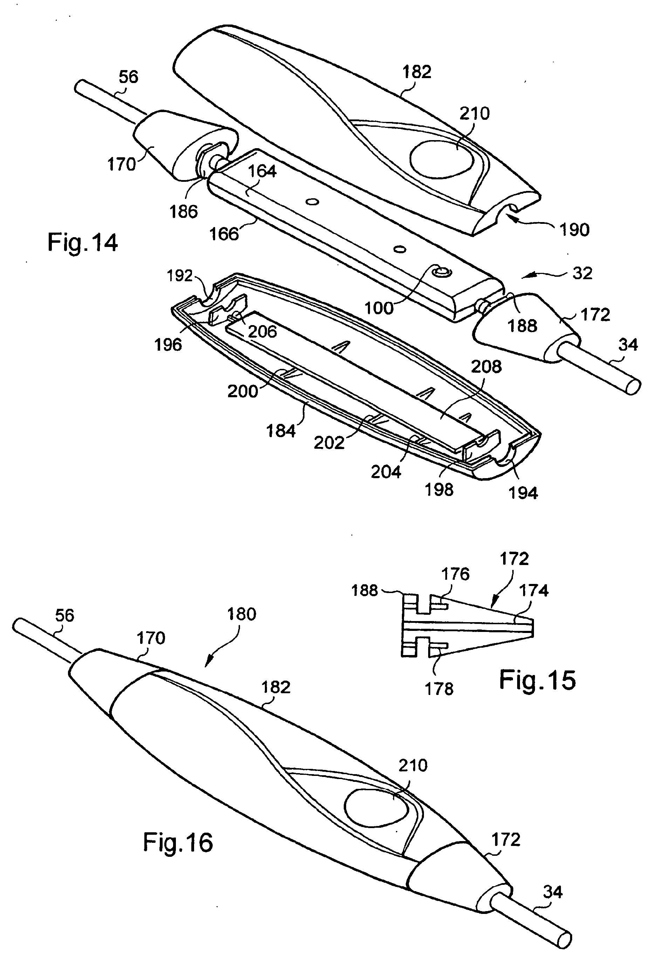

Deux manchons d'extrémités 170 et 172 sont prévus pour permettre le passage du câble correspondant 56, 34 grâce à un conduit longitudinal 174 (figure 15).

Ces manchons, tels que le manchon 172 de la figure 15, comportent des évidements longitudinaux 176, 178 qui leur confèrent une certaine souplesse. Cette souplesse permet d'éviter un cisaillement du câble pouvant se produire à la suite de manipulations et de déformations répétées auxquelles il est soumis.

Ces manchons permettent également d'assurer l'étanchéité du câble et de l'intérieur du boîtier.

Le boîtier 180 représenté à la **figure 16** et qui encapsule le module électronique 32 déjà protégé par les deux demi-coques métalliques 164 et 166 comporte deux parties en plastique formant couvercle 182, 184 (figure 14).

Ces deux parties de forme générale allongée et plus particulièrement oblongue sont assemblées l'une avec l'autre, par exemple, par collage ou par soudure aux ultrasons de manière à renfermer le module électronique 32.

On notera la présence sur chacun des manchons d'extrémité 170 et 172 d'une collerette 186, 188 (figures 14 et 15) et d'un évidement de forme au moins partiellement complémentaire pratiqué au niveau des extrémités de chacune des parties 182 et 184 formant couvercle.

Sur la **figure 14**, le demi-couvercle supérieur 182 comporte deux évidements, dont un seul 190 est représenté, et le demi-couvercle inférieur 184 comporte deux évidements 192 et 194, tous les deux apparents.

Lors de l'assemblage des demi-couvercles de part et d'autre du module électronique 32, les évidements de chaque demi-couvercle viennent s'engager entre la collerette et le corps de ce dernier de manière à rendre solidaires axialement le boîtier et les manchons d'extrémité qui prolongent ce dernier.

Les évidements aménagés au niveau des extrémités des demi-couvercles inférieur et supérieur constituent ainsi des bords de retenue des manchons correspondants.

On notera également la présence à l'intérieur du demi-couvercle inférieur 184 de parois guides transversales 196 et 198 creusées de manière à permettre le positionnement du câble correspondant.

Des nervures de rigidification transversales 200, 202 et 204, ainsi qu'une nervure longitudinale 206, dont seule une extrémité est visible, sont aménagées à l'intérieur du demi-couvercle 184 pour rigidifier ce dernier.

Une plaque 208 est agencée au fond du demi-couvercle 184 afin de permettre un bon positionnement du module électronique protégé par les deux demi-coques métalliques.

Il convient de noter que le demi-couvercle supérieur 182 comporte les mêmes agencements que ceux décrits sur le demi-couvercle 184.

L'organe d'activation 100 du détecteur n'est pas directement manipulable par l'utilisateur pour des raisons d'étanchéité et il est accessible à ce dernier par l'intermédiaire d'une zone amincie 210 prévue dans le demi-couvercle supérieur 182 et qui est adaptée à être déformée de façon locale et maîtrisée pour s'enfoncer sous la pression du doigts d'un utilisateur et revenir à sa position initiale lorsque la pression n'est plus exercée.

Selon une variante de réalisation, on remplace la zone amincie par un bouton rapporté réalisé dans une matière souple, auquel l'utilisateur aura accès et qui sera fixé au demi-couvercle 182, par exemple par collage, de façon à assurer l'étanchéité et à ne pas présenter de zones susceptibles de provoquer l'incrustation de salissures.

On notera que le boîtier 180 renfermant le module électronique 32, ainsi que les prolongements axiaux 170 et 172 de ce boîtier présentent une surface extérieure leur permettant d'être désinfectés/stérilisés aisément et les formes de cette surface extérieure sont de nature à ne pas favoriser l'incrustation de saletés.

Par ailleurs, la surface extérieure du boîtier et de ses prolongements axiaux 170, 172 est étanche au ruissellement afin d'éviter de contaminer l'intérieur du boîtier.

L'appareil de radiologie dentaire selon l'invention est donc particulièrement simple de conception et très fiable. Le signal vidéo fourni à l'unité de traitement et de visualisation 54 est de très bonne qualité car le rapport signal sur bruit du détecteur a été considérablement amélioré par rapport à l'art antérieur.

D'autres variantes de réalisations à la portée de l'homme de l'art' peuvent également être envisagées pour les différents aspects de l'invention qui viennent d'être décrits.

## Revendications

1. Appareil de radiologie dentaire comportant :
- un capteur intra-buccal (14) comprenant un détecteur (22) qui comporte une matrice à pixels actifs transformant un rayonnement reçu en au moins un signal de sortie électrique analogique,
- un module électronique (32) encapsulé dans un boîtier et qui comporte au moins un organe d'activation du détecteur, le module étant relié au capteur par une liaison filaire (34) pour la transmission audit capteur d'un signal d'activation du détecteur généré dans le module et pour la transmission au module dudit au moins un signal de sortie électrique analogique, le module comportant des moyens de conversion analogique numérique (42) dudit au moins un signal de sortie électrique analogique en au moins un signal de sortie numérique,
- une unité distante de traitement (54) et de visualisation dudit au moins un signal de sortie numérique qui est reliée au module électronique par une liaison filaire destinée à assurer la transmission à l'unité dudit au moins un signal de sortie numérique, ledit appareil de radiologie dentaire étant **caractérisé par le fait que** le module électronique encapsulé (32) a un poids et des dimensions qui sont adaptés pour permettre, lors de l'utilisation de l'appareil, le maintien du capteur dans la bouche d'un patient lorsque ledit module électronique encapsulé est suspendu audit capteur, la matrice à pixels actifs étant réalisée en technologie biCMOS

2. Appareil selon la revendication 1, **caractérisé en ce que** le module électronique est à une distance comprise entre 50 cm et deux mètres du capteur.

3. Appareil selon l'une des revendications 1 à 2, **caractérisé en ce que** le module électronique encapsulé est plus proche du capteur que de l'unité de traitement et de visualisation.

4. Appareil selon l'une des revendications 1 à 3, **caractérisé en ce que** ledit au moins un organe d'activation est un bouton poussoir.

5. Appareil selon l'une des revendications 1 à 4, **caractérisé en ce que** chaque liaison filaire est un câble.

6. Appareil selon la revendication 5, **caractérisé en ce que**, chaque câble s'insérant à une de ses extrémités dans le boîtier, le module électronique est muni de dispositifs anti-traction qui sont chacun aptes à coopérer avec une extrémité de l'un des câbles de manière à empêcher le retrait du câble correspondant du boîtier sous l'action d'une traction exercée sur ledit câble.

7. Appareil selon la revendication 6, **caractérisé en ce que**, chaque câble comportant une gaine coaxiale à un faisceau de fils électriques, au droit de l'extrémité de chaque câble qui s'insère dans le boîtier la partie du faisceau de fils correspondant est solidarisée à un corps métallique d'anti-traction du dispositif anti-traction correspondant.

8. Appareil selon la revendication 7, **caractérisé en ce que** le module électronique se présente sous la forme d'un circuit imprimé de forme générale allongée le long d'un axe longitudinal et comportant, à chacune de deux extrémités opposées disposées longitudinalement, une découpe axiale ouverte vers l'extérieur du circuit pour loger suivant l'axe longitudinal un corps métallique d'anti-traction et la partie du faisceau de fils correspondant solidarisée et alignée, la découpe étant aménagée pour empêcher le retrait du corps suivant cet axe longitudinal.

9. Appareil selon la revendication 8, **caractérisé en ce que** chaque corps métallique d'anti-traction est pourvu d'éléments d'emboîtement disposés sur des faces opposées parallèles à la direction de la partie du faisceau de fils solidarisée au corps et qui coopèrent avec des éléments d'emboîtement complémentaires aménagés respectivement sur les bords opposés longitudinaux de la découpe correspondante.

10. Appareil selon l'une des revendications 7 à 9, **caractérisé en ce que** chaque partie de chacun des faisceaux de fils solidarisée à un corps d'anti-traction est solidarisée à un fût cylindrique qui, d'une part, entoure cette dernière et, d'autre part, est solidaire du corps correspondant.

11. Appareil selon l'une des revendications 7 à 10, **caractérisé en ce que** chaque partie de chacun des faisceaux de fils solidarisée à un corps d'anti-traction est soudée directement ou indirectement à ce dernier.

12. Appareil selon l'une des revendications 8 à 11, lorsque les revendications 10 et 11 dépendent de la revendication 8, **caractérisé en ce que** deux demi-coques métalliques sont agencées de part et d'autre du circuit imprimé et assemblées l'une avec l'autre de manière à emprisonner ledit circuit imprimé.

13. Appareil selon l'une des revendications 1 à 12, **caractérisé en ce que** le boîtier comporte au moins deux parties en plastique formant couvercle et qui sont assemblées l'une avec l'autre de manière à encapsuler le module électronique.

14. Appareil selon l'une des revendications 1 à 13, **caractérisé en ce que** le boîtier présente une surface extérieure désinfectable.

15. Appareil selon la revendication 14, **caractérisé en ce que** les formes de la surface extérieure sont de nature à ne pas favoriser l'incrustation de saletés.

16. Appareil selon la revendication 14 ou 15, **caractérisé en ce que** la surface extérieure est étanche au ruissellement.

17. Appareil selon l'une des revendications 1 à 16, **caractérisé en ce que** la liaison filaire entre le module électronique et l'unité de traitement et de visualisation est conforme à la norme USB2.0.

18. Appareil selon l'une des revendications 1 à 17, **caractérisé en ce que** le capteur comporte un convertisseur de rayonnement X qui est apte à convertir en rayonnement visible un rayonnement X ayant irradié une dent.

19. Appareil selon la revendication 18, **caractérisé en ce que** le détecteur biCMOS comprenant la matrice à pixels actifs est apte à transformer au moins une partie du rayonnement visible issu de la conversion du rayonnement X en au moins un signal de sortie électrique analogique.

## Claims

1. Dental radiology apparatus, comprising:
- an intraoral sensor (44) comprising a detector (22) that includes an active pixel array converting received x-rays into at least one analog electrical output signal,
- an electronic module (32) encapsulated in a case and which has at least one detector activation device, the module being linked to the sensor by a wire link (34) for the transmission to said sensor of a detector activation signal generated in the module and for the transmission to the module of said at least one analog electrical output signal, the module having analog-digital conversion means (42) of said at least one analog electrical output signal into at least one digital output signal,
- a remote processing and display unit (54) of said at least one digital output signal which is linked to the electronic module by a wire link intended to ensure the transmission to the unit of said at least one digital output signal, said dental radiology apparatus being **characterized in that** the encapsulated electronic module (32) has weight and dimensions suited to enable, when the apparatus is used, the sensor to be held in a patient's mouth when said encapsulated electronic module is suspended from said sensor, the active pixel array being produced using biCMOS technology.

2. Apparatus according to claim 1, **characterized in that** the electronic module is at a distance of between 50 cm and two meters from the sensor.

3. Apparatus according to one of the claims 1 to 2, **characterized in that** the encapsulated electronic module is nearer the sensor than the processing and display unit.

4. Apparatus according to one of the claims 1 to 3, **characterized in that** said at least one activation device is a pushbutton.

5. Apparatus according to one of the claims 1 to 4, **characterized in that** each wire link is a cable.

6. Apparatus according to claim 5, **characterized in that**, each cable being inserted at one of its ends into the case, the electronic module is fitted with antipull devices that are each capable of working together with one end of one of the cables to prevent the removal of the corresponding cable from the case by pulling action exerted on said cable.

7. Apparatus according to claim 6, **characterized in that**, each cable having a coaxial sheath with a bundle of electrical wires, opposite the end of each cable that inserts into the case, the part of the corresponding wire bundle is made solid with a metal anti-pull body of the corresponding anti-pull device.

8. Apparatus according to claim 7, **characterized in that** the electronic module comes in the form of a printed circuit board with overall elongated shape along a longitudinal axis and having, at each of the two opposite ends arranged longitudinally, an axial cut-out open to the outside of the circuit to house in the longitudinal direction a metal anti-pull body and the part of the corresponding wire bundle made solid and aligned, the cut-out being made to prevent removal of the body in this longitudinal direction.

9. Apparatus according to claim 8, **characterized in that** each metal antipull body is provided with fitting elements arranged on the opposite sides parallel to the direction of the part of the wire bundle made solid with the body and which work together with the complementary fitting elements respectively arranged on the opposite longitudinal edges of the corresponding cut-out.

10. Apparatus according to one of the claims 7 to 9, **characterized in that** each part of each of the wire bundles made solid with an anti-pull body is made solid with a cylindrical drum that, on the one hand, surrounds the latter and, on the other hand, is held solid with the corresponding body.

11. Apparatus according to one of the claims 7 to 10, **characterized in that** each part of each of the wire bundles made solid with an anti-pull body is welded directly or indirectly to the latter.

12. Apparatus according to one of the claims 8 to 11, when claims 10 and 11 depend on claim 8, **characterized in that** two metal half-shells are arranged on either side of the printed circuit board and assembled together so as to shield said printed circuit board.

13. Apparatus according to one of the claims 1 to 12, **characterized in that** the case has at least two plastic parts forming a cover and which are assembled together to encapsulate the electronic module.

14. Apparatus according to one of the claims 1 to 13, **characterized in that** the case has an external surface that can be disinfected.

15. Apparatus according to claim 14, **characterized in that** the shapes of the external surface are of a kind not to encourage the incrustation of dirt.

16. Apparatus according to claim 14 or 15, **characterized in that** the external surface is drip-proof.

17. Apparatus according to one of the claim 1 to 16, **characterized in that** the wire link between the electronic module and the processing and display unit complies with standard USB2.0.

18. Apparatus according to one of the claims 1 to 17, **characterized in that** the sensor has an x-ray converter that is capable of converting the x-rays that have passed through a tooth into visible radiation.

19. Apparatus according to claim 18, **characterized in that** the biCMOS detector including the active pixel array is capable of converting at least one part of the visible radiation coming from the conversion of the x-rays into at least one analog electrical output signal.

## Patentansprüche

1. Zahnärztliche Röntgenvorrichtung, die umfasst:
- einen intrabukkalen Sensor (14), der einen Detektor (22) aufweist, der eine Matrix aus aktiven Pixeln enthält, die eine empfangene Strahlung in wenigstens ein analoges elektrisches Ausgangssignal transformieren;
- ein elektronisches Modul (32), das in ein Gehäuse eingekapselt ist und das wenigstens ein Organ zum Aktivieren des Detektors aufweist, wobei das Modul mit dem Sensor durch eine Drahtverbindung (34) für die Übertragung eines in dem Modul erzeugten Signals zum Aktivieren des Detektors an den Sensor und für die Übertragung des wenigstens einen analogen elektrischen Ausgangssignals an das Modul verbunden ist, wobei das Modul Mittel (42) für eine Analog/Digital-Umsetzung des wenigstens einen analogen elektrischen Ausgangssignals in wenigstens ein digitales Ausgangssignal aufweist,
- eine entfernte Verarbeitungs- und Anzeigeeinheit (54) für das wenigstens eine digitale Ausgangssignal, die mit dem elektronischen Modul durch eine Drahtverbindung verbunden ist, die dazu vorgesehen ist, die Übertragung des wenigstens einen digitalen Ausgangssignals an die Einheit zu gewährleisten, wobei die zahnärztliche Röntgenvorrichtung **dadurch gekennzeichnet ist, dass** das eingekapselte elektronische Modul (32) ein Gewicht und Abmessungen hat, die so ausgelegt sind, dass ermöglicht wird, dass bei Verwendung der Vorrichtung der Sensor im Mund eines Patienten gehalten werden kann, wenn das eingekapselte elektronische Modul an dem Sensor aufgehängt ist, wobei die Matrix aus aktiven Pixeln in BiCMOS-Technologie verwirklicht ist.

2. Vorrichtung nach Anspruch 1, **dadurch gekennzeichnet, dass** sich das elektronische Modul in einem Abstand im Bereich von 50 cm bis 2 Meter von dem Sensor befindet.

3. Vorrichtung nach einem der Ansprüche 1 bis 2, **dadurch gekennzeichnet, dass** sich das eingekapselte elektronische Modul näher bei dem Sensor als die Verarbeitungs- und Anzeigeeinheit befindet.

4. Vorrichtung nach einem der Ansprüche 1 bis 3, **dadurch gekennzeichnet, dass** das wenigstens eine Aktivierungsorgan ein Druckknopf ist.

5. Vorrichtung nach einem der Ansprüche 1 bis 4, **dadurch gekennzeichnet, dass** jede Drahtverbindung ein Kabel ist.

6. Vorrichtung nach Anspruch 5, **dadurch gekennzeichnet, dass** dann, wenn jedes Kabel mit einem seiner Enden in das Gehäuse eingesteckt ist, das elektronische Modul mit Zugverhinderungsvorrichtungen versehen ist, die jeweils so beschaffen sind, dass sie mit einem Ende eines der Kabel in der Weise zusammenwirken, dass ein Herausziehen des entsprechenden Kabels aus dem Gehäuse unter der Wirkung eines auf das Kabel ausgeübten Zugs verhindert wird.

7. Vorrichtung nach Anspruch 6, **dadurch gekennzeichnet, dass** dann, wenn jedes Kabel eine koaxiale Hülle mit einem Bündel elektrischer Drähte enthält, am Ort des Endes jedes Kabels, das in das Gehäuse eingesteckt ist, der entsprechende Teil des Bündels von Drähten mit einem metallischen Zugverhinderungskörper der entsprechenden Zugverhinderungsvorrichtung fest verbunden ist.

8. Vorrichtung nach Anspruch 7, **dadurch gekennzeichnet, dass** das elektronische Modul die Form einer gedruckten Schaltung mit einer längs einer longitudinalen Achse lang gestreckten allgemeinem Form aufweist und an jedem seiner zwei gegenüberliegenden longitudinalen Enden einen zur äußeren Umgebung der Schaltung offenen axialen Ausschnitt aufweist, um in Richtung der Längsachse einen metallischen Zugverhinderungskörper und den befestigten und ausgerichteten Teil des entsprechenden Drahtbündels aufzunehmen, wobei der Ausschnitt so beschaffen ist, dass er ein Zurückziehen des Körpers längs dieser longitudinalen Achse verhindert.

9. Vorrichtung nach Anspruch 8, **dadurch gekennzeichnet, dass** jeder metallische Zugverhinderungskörper mit Einfügungselementen versehen ist, die an gegenüberliegenden Seiten, die zu der Richtung des mit dem Körper fest verbundenen Teils des Drahtbündels parallel sind, angeordnet sind und mit komplementären Einfügungselementen, die an den jeweiligen gegenüberliegenden longitudinalen Rändern des entsprechenden Ausschnitts ausgebildet sind, zusammenwirken.

10. Vorrichtung nach einem der Ansprüche 7 bis 9, **dadurch gekennzeichnet, dass** jeder Teil jedes der Drahtbündel, der mit einem Zugverhinderungskörper fest verbunden ist, mit einem zylindrischen Schaft fest verbunden ist, der einerseits diesen letzteren umgibt und andererseits mit dem entsprechenden Körper fest verbunden ist.

11. Vorrichtung nach einem der Ansprüche 7 bis 10, **dadurch gekennzeichnet, dass** jeder Teil jedes der Drahtbündel, der mit einem Zugverhinderungskörper fest verbunden ist, direkt oder indirekt an diesen letzteren angeschweißt ist.

12. Vorrichtung nach einem der Ansprüche 8 bis 11, wenn die Ansprüche 10 und 11 von Anspruch 8 abhängen, **dadurch gekennzeichnet, dass** zwei metallische Halbschalen beiderseits der gedruckten Schaltung angeordnet und in der Weise zusammengefügt sind, dass sie die gedruckte Schaltung einschließen.

13. Vorrichtung nach einem der Ansprüche 1 bis 12, **dadurch gekennzeichnet, dass** das Gehäuse wenigstens zwei Kunststoffteile umfasst, die einen Deckel bilden und in der Weise zusammengefügt sind, dass sie das elektronische Modul einkapseln.

14. Vorrichtung nach einem der Ansprüche 1 bis 13, **dadurch gekennzeichnet, dass** das Gehäuse eine desinfizierbare äußere Oberfläche aufweist.

15. Vorrichtung nach Anspruch 14, **dadurch gekennzeichnet, dass** die Formen der äußeren Oberfläche von einer Art sind, die die Ablagerung von Schmutz nicht begünstigt.

16. Vorrichtung nach Anspruch 14 oder 15, **dadurch gekennzeichnet, dass** die äußere Oberfläche gegenüber Oberflächenwasser dicht ist.

17. Vorrichtung nach einem der Ansprüche 1 bis 16, **dadurch gekennzeichnet, dass** die Drahtverbindung zwischen dem elektronischen Modul und der Verarbeitungs- und Anzeigeeinheit gemäß der Norm USB2.0 beschaffen ist.

18. Vorrichtung nach einem der Ansprüche 1 bis 17, **dadurch gekennzeichnet, dass** der Sensor einen Röntgenstrahlungswandler aufweist, der Röntgenstrahlung, die einen Zahn bestrahlt hat, in sichtbare Strahlung umwandeln kann.

19. Vorrichtung nach Anspruch 18, **dadurch gekennzeichnet, dass** der BiCMOS-Detektor, der die Matrix aus aktiven Pixeln enthält, wenigstens einen Teil der aus der Röntgenstrahlumwandlung hervorgehenden sichtbaren Strahlung in wenigstens ein analoges elektrisches Ausgangssignal transformieren kann.
